# EUROPEAN PATENT SPECIFICATION

(11) **EP 3 520 703 B1**
(45) Date of publication and mention of the grant of the patent: **01.05.2024**
(21) Application number: 18194350.7
(22) Date of filing: 13.09.2018
(51) Int. Cl.: A61B 8/08

(54) **ULTRASOUND DIAGNOSIS APPARATUS AND COMPUTER-READABLE RECORDING MEDIUM STORING A PROGRAM FOR EXECUTING A METHOD OF OPERATING SAME**
ULTRASCHALLDIAGNOSEVORRICHTUNG UND COMPUTERLESBARES AUFZEICHNUNGSMEDIUM, DAS EIN PROGRAMM ZUR AUSFÜHRUNG EINES VERFAHRENS ZUM BETRIEB DAVON SPEICHERT
APPAREIL DE DIAGNOSTIC PAR ULTRASONS ET SUPPORT D'ENREGISTREMENT LISIBLE PAR ORDINATEUR STOCKANT UN PROGRAMME POUR L'EXÉCUTION D'UNE MÉTHODE D'EXPLOITATION DE CELUI-CI

(30) Priority: 02.02.2018 KR 20180013432
(43) Date of publication of application: 07.08.2019
(73) Proprietor: Samsung Medison Co., Ltd., Hongcheon-gun, Gangwon-do, 25108 (KR)
(72) Inventor: LEE, Jin-yong, Gyeonggi-do (KR); KIM, Jong-sik, Gyeonggi-do (KR)
(74) Representative: Arnold & Siedsma

(56) References cited:
- US-A1- 2013 006 111
- US-A1- 2016 199 029
- T. KOGA ET AL: "A new and simple Doppler method for measurement of fetal cardiac isovolumetric contraction time : Fetal isovolumetric contraction time", ULTRASOUND IN OBSTETRICS AND GYNECOLOGY, vol. 18, no. 3, 1 September 2001 (2001-09-01), pages 264-267, XP055582551, GB ISSN: 0960-7692, DOI: 10.1046/j.0960-7692.2001.00462.x
- CHEN JIAO ET AL: "Right Heart Function of Fetuses and Infants with Large Ventricular Septal Defect: A Longitudinal Case-Control Study", PEDIATRIC CARDIOLOGY, SPRINGER NEW YORK LLC, US, vol. 37, no. 8, 25 August 2016 (2016-08-25), pages 1488-1497, XP036100341, ISSN: 0172-0643, DOI: 10.1007/S00246-016-1462-Z [retrieved on 2016-08-25]
- NAWAYTOU HYTHEM M ET AL: "Right Ventricular Systolic-to-Diastolic Time Index: Hypoplastic Left Heart Fetuses Differ Significantly from Normal Fetuses", JOURNAL OF THE AMERICAN SOCIETY OF ECHOCARDIOGRAPHY, MOSBY-YEAR BOOK, INC. ST. LOUIS, MO, US, vol. 29, no. 2, 26 September 2015 (2015-09-26), pages 143-149, XP029406958, ISSN: 0894-7317, DOI: 10.1016/J.ECHO.2015.08.014
- BUI YEN K ET AL: "Tissue Doppler Is More Sensitive and Reproducible than Spectral Pulsed-Wave Doppler for Fetal Right Ventricle Myocardial Performance Index Determination in Normal and Diabetic Pregnancies", JOURNAL OF THE AMERICAN SOCIETY OF ECHOCARDIOGRAPHY, MOSBY-YEAR BOOK, INC. ST. LOUIS, MO, US, vol. 26, no. 5, 15 March 2013 (2013-03-15) , pages 507-514, XP028585753, ISSN: 0894-7317, DOI: 10.1016/J.ECHO.2013.02.006
- E. HERNANDEZ-ANDRADE ET AL: "A modified myocardial performance (Tei) index based on the use of valve clicks improves reproducibility of fetal left cardiac function assessment : Reproducibility of a modified fetal myocardial performance index", ULTRASOUND IN OBSTETRICS AND GYNECOLOGY, vol. 26, no. 3, 22 August 2005 (2005-08-22), pages 227-232, XP055583186, GB ISSN: 0960-7692, DOI: 10.1002/uog.1959

## Description

### BACKGROUND

### 1. Field

The disclosure relates to ultrasound diagnosis apparatuses and methods of operating the same.

### 2. Description of Related Art

Ultrasound diagnosis apparatuses transmit ultrasound signals generated by transducers of a probe to an object and receive information about echo signals reflected from the object, thereby obtaining an image of an internal part of the object. In particular, ultrasound diagnosis apparatuses are used for medical purposes including observing an internal area of an object, detecting foreign substances, and assessing injuries. Such ultrasound diagnosis apparatuses exhibit high stability, display images in real time, and are safe due to there being no radiation exposure, compared to diagnostic X-ray apparatuses. Therefore, an ultrasound diagnosis apparatus is widely used together with other types of imaging diagnosis apparatuses.

Alternatively or additionally, reference is made here to T. KOGA ET AL: "A new and simple Doppler method for measurement of fetal cardiac isovolumetric contraction time : Fetal isovolumetric contraction time", ULTRASOUND IN OBSTETRICS AND GYNECOLOGY, vol. 18, no. 3, 1 September 2001 (2001-09-01), pages 264-267, XP055582551, GB, ISSN: 0960-7692, DOI: 10.1046/j.0960-7692.2001.00462.x. Relative thereto, features defined at least in characterizing portions of appended independent claims are novel.

### SUMMARY

Provided are non-transitory computer-readable recording media storing programs for executing methods of operating ultrasound diagnosis and ultrasound diagnosis apparatuses that are used to further facilitate ultrasound diagnosis.

Additional aspects will be set forth in part in the description which follows and, in part, will be apparent from the description, or may be learned by practice of the presented embodiments.

### Brief Summary of the Invention

In accordance with an aspect of the disclosure, a non-transitory computer-readable recording medium storing a program for executing a method of operating an ultrasound diagnosis apparatus includes features of the appended independent claim 1.

In accordance with another aspect of the disclosure, an ultrasound diagnosis apparatus for performing diagnosis with respect to a heart of a fetus includes features of the appended independent apparatus claim.

### Outline of the technology

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating an ultrasound diagnosis apparatus according to an embodiment;
FIGS. 2A, 2B, and 2C are diagrams respectively illustrating ultrasound diagnosis apparatuses according to an embodiment;
FIG. 3 is a block diagram of a configuration of an ultrasound diagnosis apparatus according to an embodiment;
FIG. 4 is a block diagram of a configuration of an ultrasound diagnosis apparatus according to another embodiment;
FIG. 5 is a flowchart of a method of operating an ultrasound diagnosis apparatus, according to an embodiment;
FIGS. 6A and 6B show images obtained by changing a setting range according to a target area being examined;
FIGS. 7A and 7B show images obtained by an ultrasound diagnosis apparatus by adjusting a setting range;
FIG. 8 shows an image for explaining a method, performed by an ultrasound diagnosis apparatus, of acquiring Doppler data with respect to a heart of an object;
FIGS. 9A through 9D are images for explaining a first resulting image provided by an ultrasound diagnosis apparatus, according to an embodiment;
FIG. 10 is a detailed flowchart of a method of operating an ultrasound diagnosis apparatus, according to an embodiment;
FIG. 11 is a detailed flowchart of a method of operating an ultrasound diagnosis apparatus, according to an embodiment;
FIG. 12 is a block diagram of a configuration of an ultrasound diagnosis apparatus according to another embodiment; and
FIG. 13 is a detailed flowchart of a method of operating an ultrasound diagnosis apparatus, according to an embodiment.

### DETAILED DESCRIPTION

Hereinafter, the terms used in the specification will be briefly described, and then the present invention will be described in detail. The terms used in this specification are those general terms currently widely used in the art in consideration of functions regarding the present invention, but the terms may vary according to the intention of those of ordinary skill in the art, precedents, or new technology in the art. Also, specified terms may be selected by the applicant, and in this case, the detailed meaning thereof will be described in the detailed description of the invention. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Also, the term "unit" in the specification means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with a smaller number of components and "units", or may be divided into additional components and "units".

It will be understood that, although the terms "first", "second", etc. may be used herein to describe various elements and/or components, these elements and/or components should not be limited by these terms. These terms are only used to distinguish one element or component from another element or component. For example, a first element or component may be termed a second element or component or vice versa without departing from the teachings of embodiments. As used herein, the term "and/or" includes any and all combinations of one or more of the associated listed items. Expressions such as "at least one of," when preceding a list of elements, modify the entire list of elements and do not modify the individual elements of the list.

Throughout the specification, an "image" may mean multi-dimensional data formed of discrete image elements (e.g., pixels in a two-dimensional (2D) image and voxels in a three-dimensional (3D) image).

Throughout the specification, an "ultrasound image" refers to an image of an object, which is obtained using ultrasound waves. An ultrasound image may be an image obtained by transmitting ultrasound signals generated by transducers of a probe to an object and receiving information about echo signals reflected from the object. Furthermore, an ultrasound image may take different forms. For example, the ultrasound image may be at least one of an amplitude (A) mode image, a brightness (B) mode image, a color (C) mode image, and a Doppler (D) mode image. In addition, an ultrasound image may be a 2D or 3D image.

Furthermore, an "object" may be a human, an animal, or a part of a human or animal. For example, the object may be an organ (e.g., the liver, the heart, the womb, the brain, a breast, or the abdomen), a blood vessel, or a combination thereof. Furthermore, the object may be a phantom. The phantom means a material having a density, an effective atomic number, and a volume that are approximately the same as those of an organism. For example, the phantom may be a spherical phantom having properties similar to a human body.

Furthermore, throughout the specification, a "user" may be, but is not limited to, a medical expert, such as a medical doctor, a nurse, a medical laboratory technologist, a medical image expert, or a technician who repairs a medical apparatus

Embodiments will be described more fully hereinafter with reference to the accompanying drawings so that they may be easily implemented by one of ordinary skill in the art. However, the embodiments may have different forms and should not be construed as being limited to the descriptions set forth herein.

Embodiments of the invention now will be described more fully hereinafter with reference to the accompanying drawings, in which illustrative embodiments of the invention are shown.

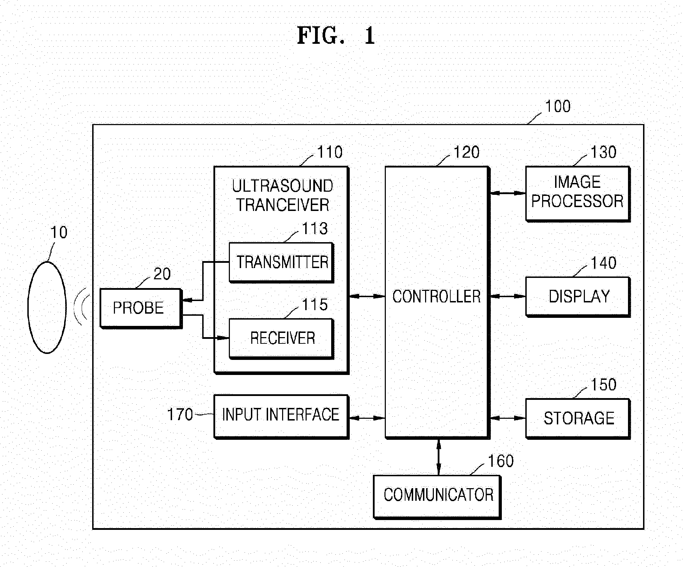

FIG. 1 is a block diagram illustrating a configuration of an ultrasound diagnosis apparatus 100, i.e., a diagnostic apparatus, according to an embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus, that is portable, moveable, mobile, or hand-held. Examples of a portable-type ultrasound diagnosis apparatus may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an embodiment is not limited thereto.

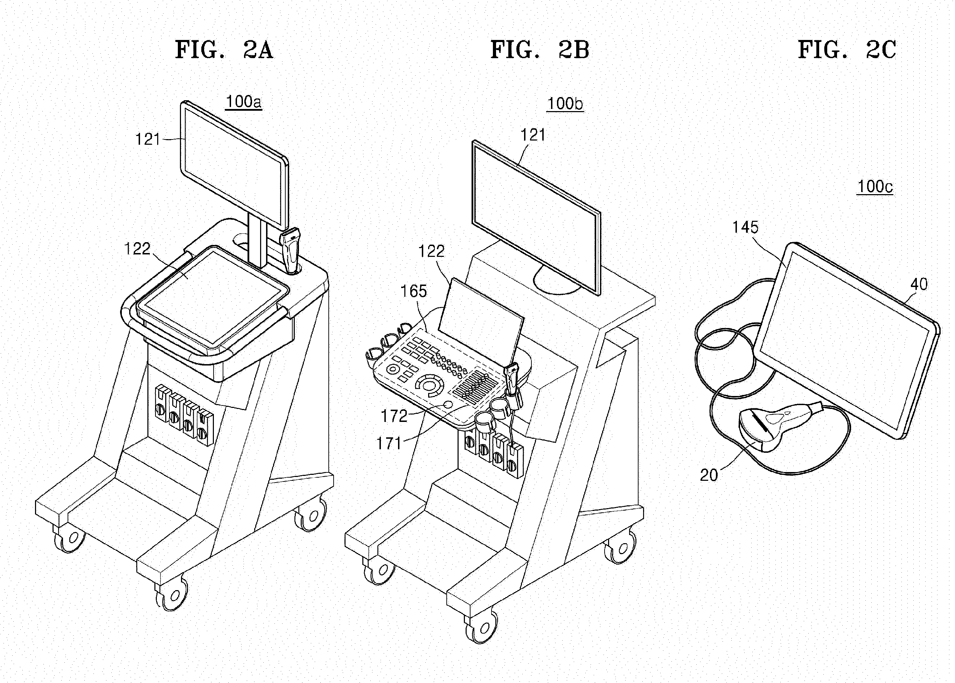

An example of the ultrasound diagnosis apparatus 100 according to the present embodiment is described below with reference to FIGS. 2A, 2B, and 2C.

FIGS. 2A, 2B, and 2C are diagrams illustrating ultrasound diagnosis apparatuses 100a, 100b, and 100c according to an embodiment.

Referring to FIGS. 2A and 2B, the ultrasound diagnosis apparatus 100a may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100a. The main display 121 and the sub-display 122 may provide graphical user interfaces (GUI), thereby receiving user's inputs of data to control the ultrasound diagnosis apparatus 100a. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100a may control the display of the ultrasound image on the main display 121 by using the input control data.

Referring to FIG. 2B, the ultrasound diagnosis apparatus 100b may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100c may be implemented as a portable ultrasound diagnosis apparatus. An example of the portable ultrasound diagnosis apparatus may include, for example, smart phones including probes and applications, laptop computers, personal digital assistants (PDAs), or tablet PCs, but an embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

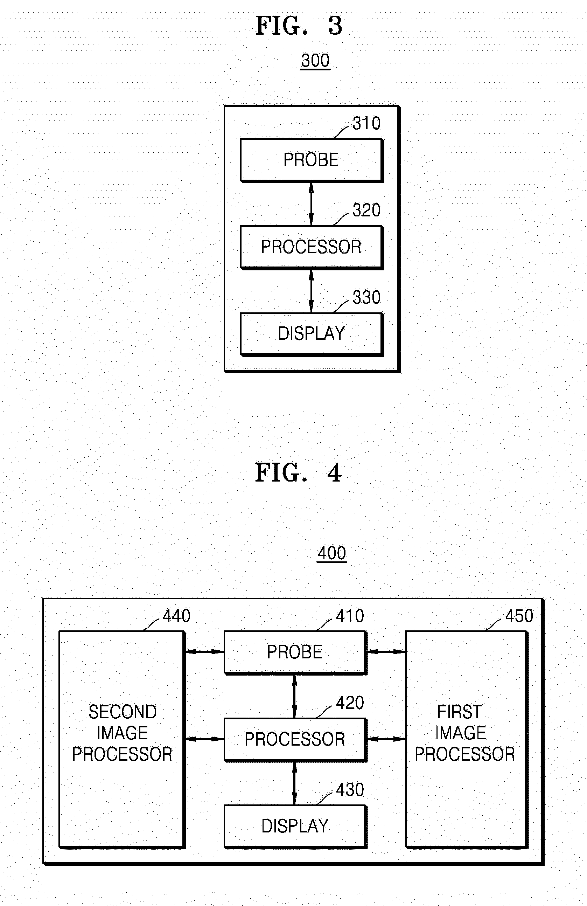

FIG. 3 is a block diagram of a configuration of an ultrasound diagnosis apparatus 300 according to an embodiment.

Referring to FIG. 3, the ultrasound diagnosis apparatus 300 according to the present embodiment may include a probe 310, a processor 320, and a display 330. However, all of the components shown in FIG. 3 are not essential components. The ultrasound diagnosis apparatus 300 may include more or fewer components than those shown in FIG. 3. Configurations and operations of the probe 310, the processor 320, and the display 330 will now be described in detail.

The probe 310 may include a plurality of transducers that convert ultrasound signals into electrical signals or vice versa. In other words, the probe 310 may include a transducer array consisting of a plurality of transducers. The plurality of transducers may be arranged in a one-dimensional (1D) or 2D array, and each of the plurality of transducers generates ultrasound signals separately or simultaneously. An ultrasound signal transmitted by each transducer is reflected off a discontinuous impedance surface within an object. Each transducer may convert a received reflected echo signal into an electrical reception signal. The probe 310 may transmit ultrasound signals to the heart of an object and receive echo signals reflected therefrom. In this case, the object may be a human. In detail, the object may include an adult, a child, a fetus, etc.

The processor 320 may receive an echo signal from the probe 310. The processor 320 may then acquire first Doppler data with respect to a first setting range based on the received echo signal. Furthermore, the processor 320 may acquire second Doppler data with respect to a second setting range based on the echo signal. In this case, the first Doppler data may refer to data regarding blood flow of the heart, and the second Doppler data may refer to data regarding valves of the heart.

The processor 320 may acquire data regarding a first resulting image to be displayed on the display 330. The processor 320 may acquire the data regarding the first resulting image based on the first Doppler data and the second Doppler data. For example, the processor 320 may acquire data regarding the first resulting image by using a difference between the first Doppler data and the second Doppler data. The processor 320 may then transmit the data regarding the first resulting image to the display 330.

The display 330 displays a predetermined screen. In detail, the display 330 may display a predetermined screen according to control by the processor 320. The display 330 includes a display panel (not shown) on which a resulting image (e.g., an ultrasound image or diagnostic image), etc. may be displayed.

The ultrasound diagnosis apparatus 300 may include a central arithmetic processor that controls all operations of the probe 310, the processor 320, and the display 330. The central arithmetic processor may be implemented as an array of a plurality of logic gates or a combination of a general purpose microprocessor and a memory for storing a program that can be run on the general purpose microprocessor. Furthermore, it will be appreciated by those of ordinary skill in the art that the central arithmetic processor may be formed by different types of hardware.

FIG. 4 is a block diagram of a configuration of an ultrasound diagnosis apparatus 400 according to another embodiment.

Referring to FIG. 4, the ultrasound diagnosis apparatus 400 according to the present embodiment may include a probe 410, a processor 420, a display 430, a first image processor 450, and a second image processor 440.

Since the probe 410, the processor 420, and the display 430 included in the ultrasound diagnosis apparatus 400 of FIG. 1 respectively correspond to the probe 310, the processor 320, and the display 330 in the ultrasound diagnosis apparatus 300 described with reference to FIG. 3, descriptions that are provided above with respect to FIG. 3 will be omitted below. The ultrasound diagnosis apparatus 400 may include more or fewer components than those shown in FIG. 4.

The ultrasound diagnosis apparatus 400 may include the first and second image processors 450 and 440. The first image processor 450 may acquire first Doppler data with respect to a first setting range based on an echo signal. The second image processor 440 may acquire second Doppler data with respect to a second setting range based on the same echo signal that is used by the first image processor 450. The first and second image processors 450 and 440 may respectively process and transform the first Doppler data and the second Doppler data and transmit the results to the processor 420. Unlike in FIG. 4, the first and second image processors 450 and 440 may be included in the processor 420.

For example, the first image processor 450 may generate data regarding a first image based on the first Doppler data. For example, the first image processor 450 may generate data regarding the first image by extracting data that satisfies the first setting range among the first Doppler data.

For example, the second image processor 440 may generate data regarding a second image based on the second Doppler data. For example, the second image processor 440 may generate data regarding the second image by extracting data that satisfies the second setting range among the second Doppler data.

In this case, the first Doppler data and the second Doppler data are acquired by transforming the same echo signal or are generated therefrom. Thus, the ultrasound diagnosis apparatus 400 according to the present embodiment may generate a resulting image via one-time diagnosis, thereby making it convenient for a user to perform diagnosis.

For example, the processor 420 may extract features by comparing the data regarding the first image with the data regarding the second image. The processor 420 may generate the first resulting image by adding the extracted features to the first image. The first resulting image may be generated using other various methods.

For example, the first or second setting range may mean a range of a setting value including at least one of a gain, a sensitivity, a power, and a blood flow velocity.

The ultrasound diagnosis apparatus 400 may further include a user interface (not shown). The user interface may refer to a device via which data for controlling the ultrasound diagnosis apparatus 400 is received from the user.

The processor 420 may control the display 430 to generate and output a user interface screen for receiving a predetermined command or data from the user. The display 430 may display, on the display panel, an input screen for respectively setting landmarks corresponding to predetermined movements of the heart in the first and second Doppler data. In this case, the predetermined movements may be an opening movement of heart valves and flow of blood through the heart.

For example, the ultrasound diagnosis apparatus 400 may further include a communicator (not shown). The communicator may receive and/or transmit data from and/or to an external device. For example, the communicator may transmit synchronized first and second Doppler data to an external terminal. Furthermore, the communicator may transmit at least one piece of data related to a function of the heart to the external terminal. In this case, the external terminal may be a patient's terminal. Furthermore, the external device a server for providing may be a relay server of an application for providing health information to a patient or a server that manages medical records of the patient. The communicator may connect to a wireless probe or an external device via a communication network based on Wi-Fi or Wi-Fi Direct (WFD) technology. In detail, examples of a wireless communication network to which the communicator can connect may include, but are not limited to, Wireless LAN (WLAN), Wi-Fi, Bluetooth, ZigBee, WFD, Ultra Wideband (UWB), Infrared Data Association (IrDA), Bluetooth Low Energy (BLE), and Near Field Communication (NFC).

The ultrasound diagnosis apparatus 400 may further include a memory (not shown). The memory may store data related to an ultrasound image (e.g., an ultrasound image, ultrasound data, scan-related data, data related to diagnosis of a patient, etc.), data transmitted from an external device to the ultrasound diagnosis apparatus 400, etc. The data transmitted from the external device may include patient-related information, data necessary for diagnosis and treatment of a patient, a patient's past medical history, a medical work list corresponding to instructions regarding diagnosis on a patient, and the like.

Furthermore, the memory may store a program for executing a method of operating the ultrasound diagnosis apparatus 400. The memory may include a code representing a method of operating the ultrasound diagnosis apparatus 400.

The ultrasound diagnosis apparatus 400 may include a central arithmetic processor that controls all operations of the probe 410, the processor 420, the display 430, the first image processor 450, the second image processor 440, and the memory. The central arithmetic processor may be implemented as an array of a plurality of logic gates or a combination of a general purpose microprocessor and a memory for storing a program that can be executed on the general purpose microprocessor. Furthermore, it will be appreciated by those of ordinary skill in the art that the central arithmetic processor may be formed by different types of hardware.

Hereinafter, various operations performed by the ultrasound diagnosis apparatus 300 (400) and applications thereof will be described in detail. Although none of the probe 310 (410), the processor 320 (420), the display 330 (430), the user interface, the communicator, and the memory are specified, features and aspects that would be clearly understood by and are obvious to those of ordinary skill in the art may be considered as a typical implementation. The scope of the present disclosure is not limited by a name of a particular component or a physical/logical structure.

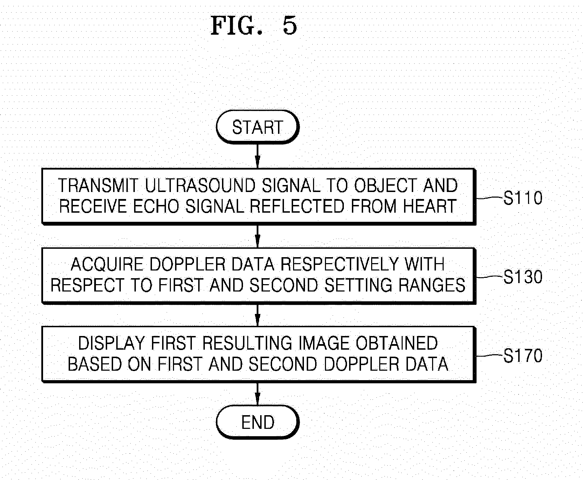

FIG. 5 is a flowchart of a method of operating an ultrasound diagnosis apparatus, according to an embodiment.

The method according to the present embodiment may include operations of: transmitting an ultrasound signal to an object and receiving an echo signal reflected from the heart (S110); acquiring first Doppler data with respect to a first setting range and second Doppler data with respect to a second setting range (S130); and displaying a first resulting image obtained based on the first and second Doppler data (S170).

For example, in operations S130 and S170, the first and second setting ranges may each be ranges of a magnitude of a signal and a velocity of blood flow. Furthermore, the first Doppler data may be data in which a magnitude of a signal is a within a first range, and a velocity of blood flow is within a second range. The second Doppler data may be data in which the magnitude of the signal is within a third range, and the velocity of blood flow is within a third range.

According to the invention, the first Doppler data are acquired with respect to the first setting range by using an echo signal. Furthermore, the second Doppler data are acquired with respect to the second setting range by using the same echo signal.

According to an embodiment, in operation S170, to obtain the first resulting image, the processor 420 may mix the first Doppler data with the second Doppler data according to a predetermined method.

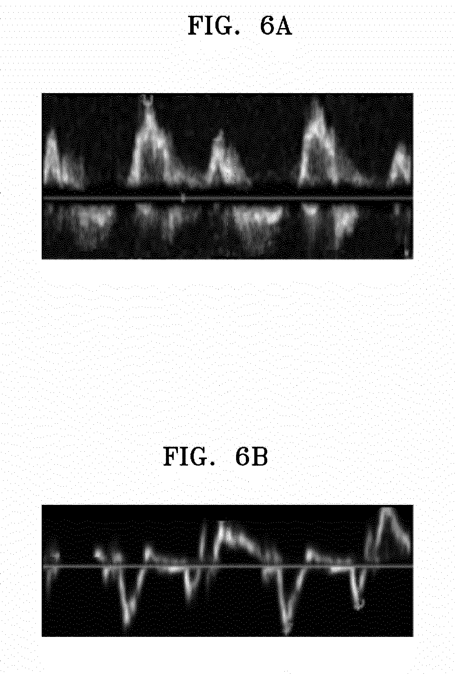

FIGS. 6A and 6B show images obtained by changing a setting range according to a target area being observed.

For example, FIG. 6A may show an image obtained by optimizing a setting range for observation of a blood flow velocity, and FIG. 6B may show an image obtained by optimizing a setting range for observation of movement of an organ. In this way, the type of an image that can be obtained by the user varies according to a setting range. However, it may be inconvenient for a user to observe a plurality of these images or individually generate and observe the images.

According to an embodiment, as shown in FIG. 4, an ultrasound diagnosis apparatus may include first and second image processors that respectively acquire pieces of Doppler data with respect to two or more setting ranges based on one ultrasound signal (or echo signal). By combining together the acquired two or more pieces of Doppler data according to a predetermined method, it is possible to provide important information to the user.

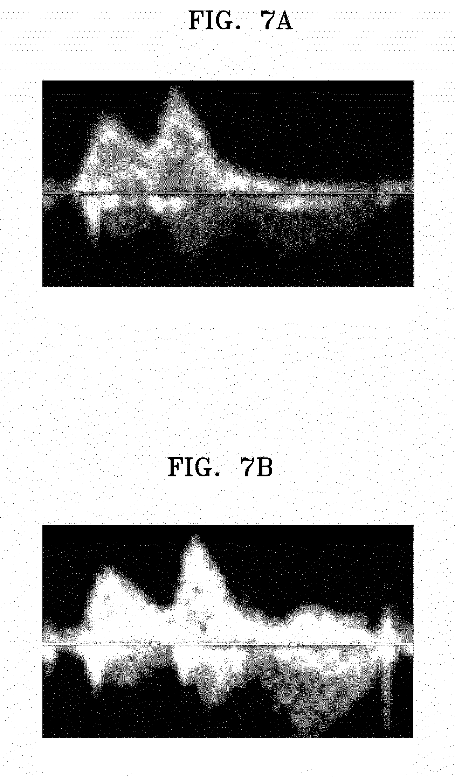

FIGS. 7A and 7B show images obtained by an ultrasound diagnosis apparatus by adjusting a setting range.

Doppler is a method of observing a blood flow velocity using ultrasound waves. Furthermore, pulsed wave Doppler or continuous wave Doppler may be generally used for relatively accurate measurement of blood flow velocity. In this case, various setting values may be used to predict a range of a blood flow velocity or obtain a user's desired image. However, use of these setting values or setting ranges may cause the user to miss important information. Furthermore, when the user changes a setting to observe more information, unimportant information may be included and thus, the user may miss actual important information.

For example, FIG. 7A may be an image obtained by generally adjusting a setting range for observation of blood flow, and FIG. 7B may be an image obtained by excessively adjusting a setting range. In other words, both general setting as shown in FIG. 7A and excessive setting as shown in FIG. 7B may make it difficult for the user to identify important information.

According to an embodiment, an ultrasound diagnosis apparatus may respectively acquire pieces of Doppler data with respect to two setting ranges and process the acquired Doppler data by using a predetermined method, thereby enabling a user to easily identify important information. According to another embodiment, an ultrasound diagnosis apparatus may respectively acquire pieces of Doppler data with respect to two or more setting ranges.

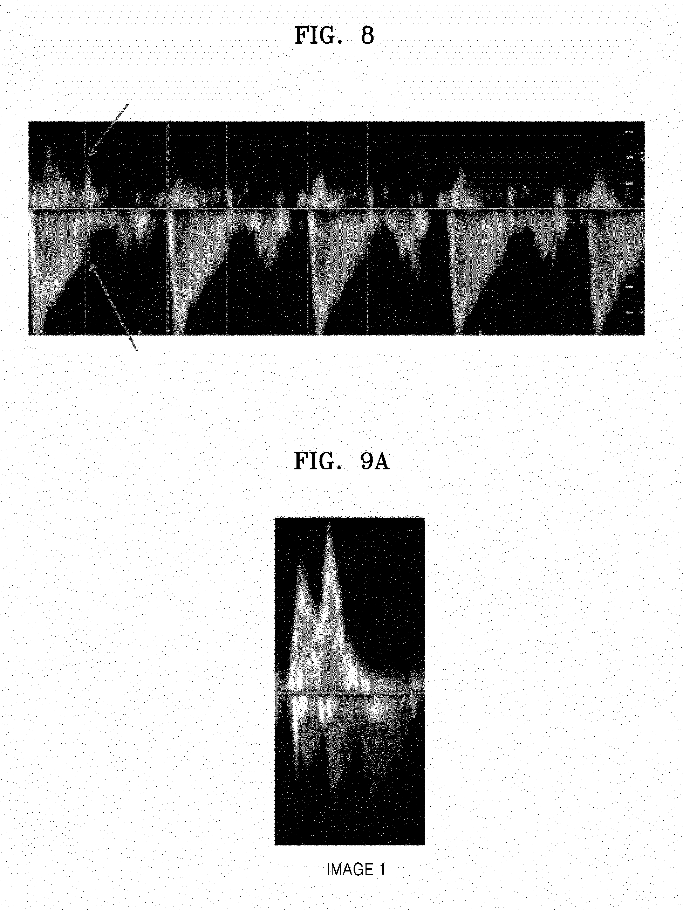

FIG. 8 shows an image for explaining a method, performed by an ultrasound diagnosis apparatus, of acquiring Doppler data with respect to a heart of an object.

A waveform shown in FIG. 8 represents a pulsed wave Doppler signal. The pulsed wave Doppler signal may be used to display a blood flow image. A click signal is indicated by arrows and may be used to detect a valve signal. However, since the click signal is not clearly identified, it may not be easy to clearly provide a valve signal to the user. Hereinafter, an example in which such a valve signal is extracted from a blood flow image and provided to the user according to an embodiment will be described.

FIGS. 9A through 9D show images for explaining a first resulting image provided by an ultrasound diagnosis apparatus, according to an embodiment.

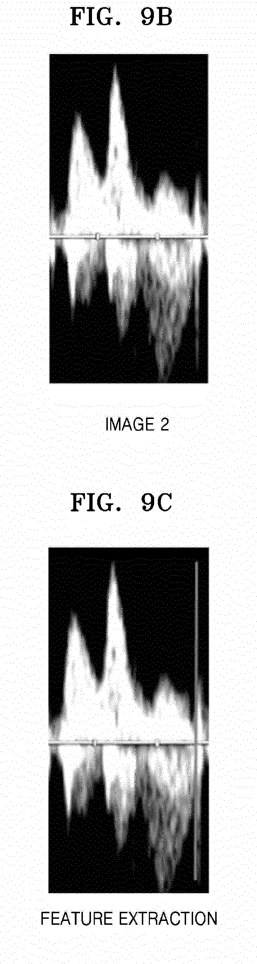

FIG. 9A illustrates a first image obtained based on first Doppler data, and FIG. 9B illustrates a second image obtained based on second Doppler data.

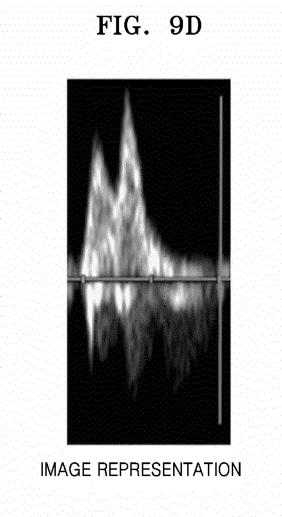

FIG. 9C illustrates a feature extraction image obtained from a second image, and FIG. 9D illustrates a first resulting image obtained by adding the feature extraction image to the first image.

For example, the processor 420 may obtain the feature extraction image of FIG. 9C from the first image of FIG. 9A and the second image of FIG. 9B. The processor 420 may also obtain the first resulting image of FIG. 9D by adding the feature extraction image of FIG. 9C to the first or second image.

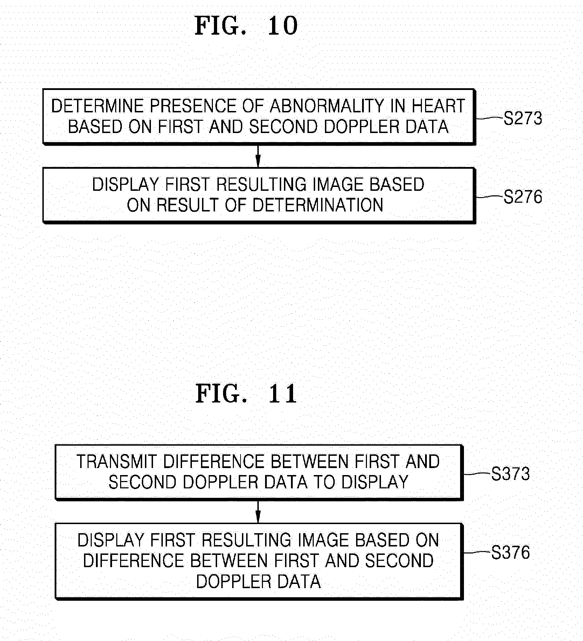

FIG. 10 is a detailed flowchart of a method of operating the ultrasound diagnosis apparatus 400, according to an embodiment.

Operation S170 described with reference to FIG. 5 may include operations S273 and S276. Referring to FIG. 10, the processor 420 may determine the presence of an abnormality in the heart based on the first and second Doppler data (S273). In this case, to do so, the processor 420 may calculate a same volume interval in a cardiac cycle of the heart. For example, the processor 420 may determine the presence of an abnormality in the heart by determining a length of the same volume interval. In detail, the processor 420 may determine the presence of an abnormality in the heart by determining whether the length of the same volume interval is less than or equal to a reference value.

The processor 420 may display a first resulting image based on a result of the determining in operation S273 (S276). For example, when any abnormality of the heart is present, the processor 420 may indicate the presence of an abnormality in the heart on the first resulting image by displaying a separate marker. For example, the processor 420 may indicate the presence of an abnormality in the heart on the first resulting image based on an input received via the user interface.

FIG. 11 is a detailed flowchart of a method of operating the ultrasound diagnosis apparatus 400, according to an embodiment.

Operation S170 described with reference to FIG. 5 may include operations S373 and S376. Referring to FIG. 11, the processor 420 may transmit a difference between the first Doppler data and the second Doppler data to the display 430 (S373). The processor 420 may then display a first resulting image based on the difference between the first and second Doppler data (S376).

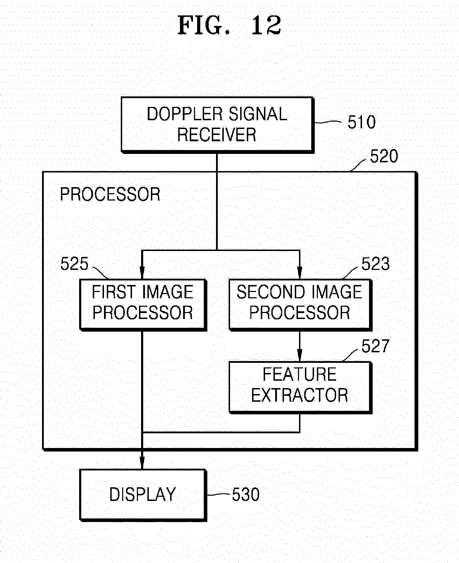

FIG. 12 is a block diagram of a configuration of an ultrasound diagnosis apparatus according to another embodiment.

Referring to FIG. 12, the ultrasound diagnosis apparatus according to the present embodiment may include a Doppler signal receiver 510, a processor 520, and a display 530. The processor 520 may include a first image generator 525, a second image generator 523, and a feature extractor 527.

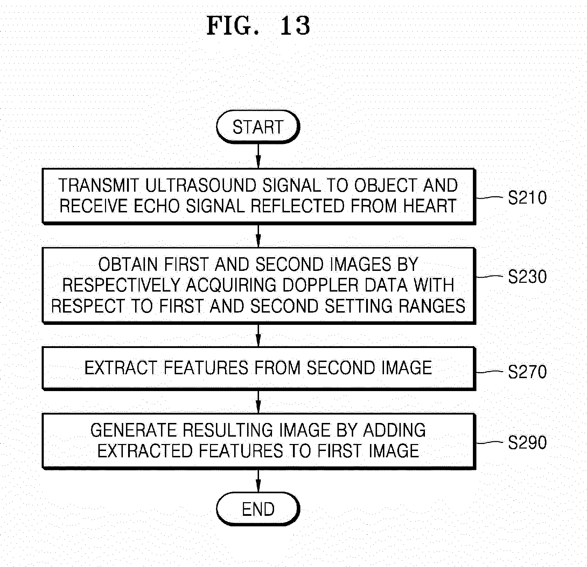

FIG. 13 is a detailed flowchart of a method of operating the ultrasound diagnosis apparatus of FIG. 12, according to an embodiment.

The Doppler signal receiver 510 may transmit an ultrasound signal to an object and receive an echo signal reflected from the heart (S210).

The first image generator 525 may obtain a first image by acquiring Doppler data with respect to a first setting range, and the second image generator 523 may obtain a second image by acquiring Doppler data with respect to a second setting range (S230).

For example, the first image generator 525 may generate first Doppler data by transforming an echo signal received from a fetus's heart only within the first setting range and obtain the first image based on the first Doppler data. The second image generator 523 may generate second Doppler data by transforming the same echo signal that is used to obtain the first image only within the second setting range and obtain the second image based on the second Doppler data.

By performing these operations, image 1 as shown in FIG. 9A and image 2 as shown in FIG. 9B may be obtained.

The feature extractor 527 may extract features from the second image (S270). By performing operation S270, a feature extraction image as shown in FIG. 9C may be obtained. The processor 520 may generate a resulting image by adding the extracted features to the first image (S290). By performing operation S290, an image representation as shown in FIG. 9D may be obtained.

According to various embodiments, the first and second images may be processed using various methods. Furthermore, the number of images being processed is not limited to two (2) but may be implemented in various ways.

According to various embodiments, the processor 520 may extract features by combining pieces of data regarding a plurality of images in various ways and generate a resulting image by combining the extracted features with differently processed data.

The ultrasound diagnosis apparatuses described above may be implemented using hardware components, software components, and/or a combination thereof. For example, the apparatuses and components illustrated in the embodiments may be implemented using one or more general-purpose or special-purpose computers, such as a processor, a controller, an arithmetic logic unit (ALU), a digital signal processor, a microcomputer, a field programmable array (FPA), a programmable logic unit (PLU), a microprocessor, or any other device capable of responding to and executing instructions.

A processing device may run an operating system (OS) and one or more software applications running on the OS. The processing device may also access, store, manipulate, process, and create data in response to execution of software.

Although a single processing device may be illustrated for convenience, one of ordinary skill in the art will appreciate that a processing device may include a plurality of processing elements and/or a plurality of types of processing elements. For example, a processing device may include a plurality of processors or a processor and a controller. In addition, the processing device may have different processing configurations such as parallel processors.

Software may include a computer program, a piece of code, an instruction, or one or more combinations thereof and independently or collectively instruct or configure the processing device to operate as desired.

Software and/or data may be embodied permanently or temporarily in any type of machine, component, physical equipment, virtual equipment, computer storage medium or device, or in a transmitted signal wave so as to be interpreted by the processing device or to provide instructions or data to the processing device. The software may also be distributed over network-coupled computer systems so that the software is stored and executed in a distributed fashion. The software and data may be stored in one or more computer-readable recording media.

The methods according to the embodiments may be recorded in non-transitory computer-readable recording media including program instructions to implement various operations embodied by a computer. The non-transitory computer-readable recording media may also include, alone or in combination with the program instructions, data files, data structures, and the like. The program instructions recorded in the non-transitory computer-readable recording media may be designed and configured specially for the embodiments or be known and available to those of ordinary skill in computer software.

Examples of non-transitory computer-readable recording media include magnetic media such as hard disks, floppy disks, and magnetic tape, optical media such as CD-ROM discs and DVDs, magneto-optical media such as floptical discs, and hardware devices that are specially configured to store and perform program instructions, such as ROM, RAM, flash memory, and the like.

Examples of program instructions include both machine code made by a compiler and a high-level programming language to be executed in the computer by using an interpreter.

The above-described hardware devices may be configured to act as one or more software modules in order to perform the operations of the above-described embodiments, or vice versa.

While one or more embodiments have been described with reference to the figures, it will be understood by those of ordinary skill in the art that various modifications and changes in form and details may be made from the above descriptions as far as they fall within the scope defined by the following claims. For example, adequate effects may be achieved even if the above techniques are performed in a different order than that described above, and/or the aforementioned elements, such as systems, structures, devices, or circuits, are combined or coupled in different forms and modes than those described above or are replaced or supplemented by other components or their equivalents.

Thus, the scope of the present disclosure is defined not by the detailed description thereof but by the appended claims.

## Claims

1. A non-transitory computer-readable recording medium storing a program for executing a method of operating an ultrasound diagnosis apparatus for performing diagnosis with respect to a heart of a fetus, the method comprising:
transmitting an ultrasound signal to the heart of the fetus and receiving an echo signal reflected from the heart (S210);
acquiring first Doppler data and second Doppler data respectively with respect to first and second setting ranges by using the echo signal (S230),
wherein the first Doppler data is data regarding blood flow of the heart, and the second Doppler data is data regarding valves of the heart;
obtaining a first image based on the first Doppler data;
obtaining a second image based on the second Doppler data;
obtaining a feature extraction image representing properties of the heart from the second image (S270);
obtaining a first resulting image by adding the feature extraction image to the first Doppler image (S290); and
displaying the first resulting image, **characterised in that**
the first and second setting ranges each include a range of a magnitude of a signal and a range of a velocity of blood flow, and
wherein the first and second Doppler data are each data in which the magnitude of the signal is within a predetermined range, and the velocity of the blood flow is within a predetermined range.

2. The computer-readable medium of claim 1, wherein the first resulting image is represented such that a user recognizes the properties of the heart.

3. An ultrasound diagnosis apparatus for performing diagnosis with respect to a heart of a fetus, the ultrasound diagnosis apparatus comprising:
a probe configured to transmit an ultrasound signal to the heart of the fetus and receive an echo signal reflected from the heart (510);
a processor (520) configured to:
respectively acquire first Doppler data and second Doppler data with respect to first and second setting ranges by using the echo signal, wherein the first Doppler data is data regarding blood flow of the heart, and the second Doppler data is data regarding valves of the heart;
obtain a first image based on the first Doppler data;
obtain a second image based on the second Doppler data;
obtain a feature extraction image representing properties of the heart from the second image;
obtain a first resulting image by adding the feature extraction image to the first Doppler image; data,
a display (530) configured to display the first resulting image, **characterised in that**
the first and second setting ranges each include a range of a magnitude of a signal and a range of a velocity of blood flow, and
wherein the first and second Doppler data are each data in which the magnitude of the signal is within a predetermined range, and the velocity of the blood flow is within a predetermined range.

## Patentansprüche

1. Nicht flüchtiges, computerlesbares Aufzeichnungsmedium, auf dem ein Programm zum Ausführen eines Verfahrens zum Betreiben einer Ultraschalldiagnosevorrichtung zum Durchführen einer Diagnose in Bezug auf ein Herz eines Fötus gespeichert ist, wobei das Verfahren Folgendes umfasst:
Übertragen eines Ultraschallsignals an das Herz des Fötus und Empfangen eines von dem Herzen reflektierten Echosignals (S210);
Erfassen von ersten Dopplerdaten und zweiten Dopplerdaten jeweils in Bezug auf erste und zweite Einstellbereiche unter Verwendung des Echosignals (S230), wobei es sich bei den ersten Dopplerdaten um Daten bezüglich des Blutflusses des Herzens und bei den zweiten Dopplerdaten um Daten bezüglich der Klappen des Herzens handelt;
Erhalten eines ersten Bildes basierend auf den ersten Dopplerdaten;
Erhalten eines zweiten Bildes basierend auf den zweiten Dopplerdaten;
Erhalten eines Merkmalsextraktionsbildes, das Eigenschaften des Herzens darstellt, und zwar aus dem zweiten Bild (S270);
Erhalten eines ersten resultierenden Bildes durch Addieren des Merkmalsextraktionsbildes zu dem ersten Dopplerbild (S290); und
Anzeigen des ersten resultierenden Bildes,
**dadurch gekennzeichnet, dass** die ersten und zweiten Einstellbereiche jeweils einen Bereich einer Größe eines Signals und einen Bereich einer Geschwindigkeit des Blutflusses enthalten, und
wobei es sich bei den ersten und zweiten Dopplerdaten jeweils um Daten handelt, bei denen die Größe des Signals innerhalb eines vorbestimmten Bereichs liegt und die Geschwindigkeit des Blutflusses innerhalb eines vorbestimmten Bereichs liegt.

2. Computerlesbares Medium nach Anspruch 1, wobei das erste resultierende Bild so dargestellt wird, dass ein Benutzer die Eigenschaften des Herzens erkennen kann.

3. Ultraschalldiagnosevorrichtung zum Durchführen einer Diagnose in Bezug auf ein Herz eines Fötus, wobei die Ultraschalldiagnosevorrichtung Folgendes umfasst:
eine Sonde, die so ausgelegt ist, dass sie ein Ultraschallsignal an das Herz des Fötus überträgt und ein von dem Herzen reflektiertes Echosignal empfängt (510);
einen Prozessor (520), der zu Folgendem ausgelegt ist:
Erfassen von ersten Dopplerdaten und zweiten Dopplerdaten jeweils in Bezug auf erste und zweite Einstellbereiche unter Verwendung des Echosignals, wobei es sich bei den ersten Dopplerdaten um Daten bezüglich des Blutflusses des Herzens und bei den zweiten Dopplerdaten um Daten bezüglich der Klappen des Herzens handelt;
Erhalten eines ersten Bildes basierend auf den ersten Dopplerdaten;
Erhalten eines zweiten Bildes basierend auf den zweiten Dopplerdaten;
Erhalten eines Merkmalsextraktionsbildes, das Eigenschaften des Herzens darstellt, und zwar aus dem zweiten Bild;
Erhalten eines ersten resultierenden Bildes durch Addieren des Merkmalsextraktionsbildes zu dem ersten Dopplerbild; und
eine Anzeige (530), die so ausgelegt ist, dass sie das erste resultierende Bild anzeigt,
**dadurch gekennzeichnet, dass** die ersten und zweiten Einstellbereiche jeweils einen Bereich einer Größe eines Signals und einen Bereich einer Geschwindigkeit des Blutflusses enthalten, und
wobei es sich bei den ersten und zweiten Dopplerdaten jeweils um Daten handelt, bei denen die Größe des Signals innerhalb eines vorbestimmten Bereichs liegt und die Geschwindigkeit des Blutflusses innerhalb eines vorbestimmten Bereichs liegt.

## Revendications

1. Support d'enregistrement non transitoire lisible par ordinateur, sur lequel est stocké un programme visant à exécuter un procédé d'exploitation d'un appareil d'échographie diagnostique permettant d'effectuer un diagnostic concernant le coeur d'un foetus, le procédé comprenant :
l'émission d'un signal ultrasonore vers le coeur du foetus et la réception d'un signal d'écho réfléchi par le coeur (S210),
l'acquisition de premières données Doppler et de deuxièmes données Doppler respectivement en lien avec des première et deuxième plages de réglage, à l'aide du signal d'écho (S230), lesdites premières données Doppler consistant en des données concernant le flux sanguin du coeur et lesdites deuxièmes données Doppler consistant en des données concernant les valvules du coeur,
l'obtention d'une première image compte tenu des premières données Doppler,
l'obtention d'une deuxième image compte tenu des deuxièmes données Doppler, l'obtention, à partir de la deuxième image, d'une image d'extraction de caractéristiques représentant les propriétés du coeur (S270),
l'obtention d'une première image résultante par addition de l'image d'extraction de caractéristiques à la première image Doppler (S290), et
l'affichage de la première image résultante ;
**caractérisé en ce que** les première et deuxième plages de réglage comprennent chacune une plage de grandeur d'un signal et une plage de vitesse de flux sanguin ;
lesdites premières et deuxièmes données Doppler consistant dans chaque cas en des données dans lesquelles la grandeur du signal se situe dans une plage prédéterminée et la vitesse du flux sanguin se situe dans une plage prédéterminée.

2. Support lisible par ordinateur selon la revendication 1, dans lequel la première image résultante est représentée de telle façon qu'un utilisateur reconnaisse les propriétés du coeur.

3. Appareil d'échographie diagnostique permettant d'effectuer un diagnostic concernant le coeur d'un foetus, l'appareil d'échographie diagnostique comprenant :
une sonde conçue pour transmettre un signal ultrasonore au coeur du foetus et recevoir un signal d'écho réfléchi par le coeur (510) ;
un processeur (520) conçu pour :
acquérir des premières données Doppler et des deuxièmes données Doppler respectivement en lien avec des première et deuxième plages de réglage à l'aide du signal d'écho, lesdites premières données Doppler consistant en des données concernant le flux sanguin du coeur et lesdites deuxièmes données Doppler consistant en des données concernant les valvules du coeur,
obtenir une première image compte tenu des premières données Doppler,
obtenir une deuxième image compte tenu des deuxièmes données Doppler, obtenir, à partir de la deuxième image, une image d'extraction de caractéristiques représentant les propriétés du coeur, et
obtenir une première image résultante par addition de l'image d'extraction de caractéristiques à la première image Doppler ; et
un écran (530) conçu pour afficher la première image résultante ;
**caractérisé en ce que** les première et deuxième plages de réglage comprennent chacune une plage de grandeur d'un signal et une plage de vitesse de flux sanguin ;
lesdites premières et deuxièmes données Doppler consistant dans chaque cas en des données dans lesquelles la grandeur du signal se situe dans une plage prédéterminée et la vitesse du flux sanguin se situe dans une plage prédéterminée.
